# EUROPÄISCHE PATENTANMELDUNG

(11) **EP 0 764 439 A2**
(43) Veröffentlichungstag der Anmeldung: **26.03.1997**
(21) Anmeldenummer: 96114597.6
(22) Anmeldetag: 12.09.1996
(51) Int. Cl.: A61K 7/48, A61K 47/44

(54) **Verwendung von Pflanzenfetten in Lebensmittelqualität als Emoillient oder Konsistenzgeber für kosmetische oder pharmazeutische Präparate**

(30) Priorität: 22.09.1995 DE 19535163
(71) Anmelder: MÄURER + WIRTZ GmbH & Co. KG, D-52224 Stolberg (DE)
(72) Erfinder: Müller, Wilfried, 52531 Übach-Palenberg (DE); Vathje, Rainer, 52223 Stolberg (DE)
(74) Vertreter: Fleischer, Holm Herbert, Dr.

(57) **Zusammenfassung**

Die vorliegende Erfindung betrifft die Verwendung eines Pflanzenfetts mit
a) einem Wassergehalt von höchstens 0,15 Gew%,
b) einem Gehalt an freien Fettsäuren von höchstens 0,15 Gew%,
c) einer Peroxidzahl nach Wheeler von höchstens 1,5 meq O₂/kg,
d) einem Steigschmelzpunkt von höchstens 45°C und
e) einer Jodzahl von höchstens 100, in dem
f) mindestens drei verschiedene an Glycerin gebundene Fettsäuren, ausgewählt aus
   Caprylsäure, Caprinsäure, Laurinsäure, Myristinsäure, Palmitinsäure, Stearinsäure, Ölsäure und Linolsäure, in einem Anteil von jeweils mindestens 5 % und gegebenenfalls weitere an Glycerin gebundene Fettsäuren in einem Anteil von jeweils höchstens 5 %, bezogen auf die Gesamtmasse der an Glycerin gebundenen Fettsäuren, vorkommen,
   sowie von Gemischen solcher Pflanzenfette als Emoillient oder Konsistenzgeber in kosmetischen oder pharmazeutischen Präparaten.

## Beschreibung

Die vorliegende Erfindung betrifft Pflanzenfette in Lebensmittelqualität, die als Emoillient und Konsistenzgeber für kosmetische oder pharmazeutische Präparate verwendet werden können.

In kosmetischen bzw. pharmazeutischen Mitteln, wie z.B. Cremes, Lotionen, Salben, Ölbäder oder Stiften werden Fettkomponenten benötigt, die eine pflegende Wirkung besitzen und gleichzeitig als Träger für lipoide Wirkstoffe wirken. Die mit diesen Fetten hergestellten Mittel dürfen nicht klebrig sein und müssen ein schnell eintretendes langanhaltendes Gefühl der Pflege vermitteln. Neben diesen taktilen Eigenschaften müssen diese Präparate geruchlich möglichst neutral sein. Zusätzlich müssen die für kosmetische bzw. pharmazeutische Zusammensetzungen verwendeten Rohstoffe bestimmten toxikologischen und ökotoxologischen Anforderungen genügen.

Wegen ihrer Verfügbarkeit und leichten Formulierbarkeit wurden bisher z.B. Paraffinöl oder Isopropylmyristat als Emoillient in kosmetischen Mitteln verwendet. Diese Ausgangsstoffe weisen aber einige entscheidende Nachteile auf. So wurden in dickflüssigen und dünnflüssigen Paraffinen aromatische polycyclische Kohlenwasserstoffe aufgefunden, von denen angenommen wird, daß sie ein gewisses carcinogenes Potential besitzen. Darüber hinaus neigen Paraffine zu Okklusivität. Isopropylmyristat auf der anderen Seite zeichnet sich durch eine erhöhte Komedonaktivität aus.

Zusätzlich zu diesen objektivbaren Gründen gibt es derzeit Tendenzen in Richtung von Produkten, die mit nachwachsenden Rohstoffen formuliert werden. Vor diesem Hintergrund besteht ein Bedürfnis, die bisher bevorzugt verwendeten Fettbestandteile auf petrochemischer und halbsynthetischer Basis durch nachwachsende Inhaltsstoffe, deren gutes toxikologisches und ökotoxikologisches Profil bekannt ist, zu ersetzen.

Für ihre hervorragenden toxikologischen und ökotoxikologischen Eigenschaften sind Triglyceride pflanzlichen Ursprungs, wie z.B. Sojabohnenöl, Palmöl und Kokosfett bekannt. Wegen ihrer Oxydationsanfälligkeit und des damit verbundenen unangenehmen Geruches werden sie üblicherweise nicht oder nur in geringer Menge in kosmetischen bzw. pharmazeutischen Mitteln verwendet, oder es müssen große Mengen an Antioxidantien zugesetzt werden. Die schlechte geruchliche Qualität wird im wesentlichen durch kurzkettige Aldehyde und Ketone und anderen Zersetzungsprodukten hervorgerufen, die auch aus dermatologischen Gründen in kosmetischen bzw. pharmazeutischen Mitteln limitiert sein sollten.

Aus diesem Grunde werden häufig synthetische Triglyceride eingesetzt, die aus destillierten Fettsäuren und Glycerin synthetisiert werden. So werden z.B. mittelkettige Triglyceride, wie Capryl/Caprinsäuretriglyceride oder Laurinsäuretriglyceride als Emoillient für die Formulierung von Emulsionen eingesetzt. Ebenso werden auch Konsistenzgeber, die höher schmelzende Triglyceride auf Basis von Myristinsäure, Stearinsäure oder Hydroxystearinsäure enthalten, verwendet. Diese Verbindungen zeichnen sich durch einen engen C-Kettenschnitt der verwendeten Fettsäuren und einem minimierten Anteil von ungesättigten Fettsäuren aus.

Diese synthetisch erzeugten Triglyceride weisen aber auch sowohl kosmetische als auch toxikologische Nachteile auf. So fühlen sich kosmetische Zusammensetzungen, die synthetisch erzeugte Triglyceride enthalten, beim Auftragen auf die Haut unangenehm an oder die Konsistenz solcher kosmetischen Produkte läßt zu wünschen übrig, so daß noch zusätzlich Wachse oder Triglyceride mit höherkettigen Fettsäuren zugesetzt werden müssen. Zu den technischen Nachteilen gehört, daß die synthetischen Triglyceride noch Rückstände aus dem jeweiligen Produktionsprozeß, wie z.B. Katalysatoren oder Schleppmittel enthalten können, die ebenfalls aus toxikologischen Gründen bedenklich sein können.

Triglyceride, die mit ungesättigten Fettsäuren wie z.B. Ölsäure, Linolsäure oder Linolensäure verestert sind, tragen zwar zur Verbesserung des Hautgefühls und des Hautzustandes bei, der hohe Anteil an ungesättigten Fettsäuren führt aber zu der bereits angesprochenen Oxidationsanfälligkeit und den damit verbundenen Geruchsproblemen. Selbst die Anwendung von Antioxidantien bringt keine ausreichende Verbesserung.

In DE-A-32 27 421 werden oxidationsstabile Mischungen von Pflanzenölen mit Jojobaöl vorgeschlagen. Diese Mischungen sind in ihrer Oxidationsstabilität allerdings abhängig von einem bestimmten Anteil des teuren Jojobaöls und beschränken sich auf Mischungen des Jojobaöls mit Haselnußöl, Mangoöl, Kaffeewachs, Karitébutter, Sojaöl, Palmöl und Maiskeimöl. Somit sind diese Mischungen sowohl aus wirtschaftlicher Hinsicht als auch aufgrund des beschränkten Anwendungsbereiches ungeeignet.

Somit besteht ein Bedürfnis, Rohstoffe für die Verwendung als Emoillient oder Konsistenzgebers in kosmetischen oder pharmazeutischen Zusammensetzungen aufzufinden, die die geschilderten Nachteile des Standes der Technik vermeiden.

Überraschenderweise wurde nun gefunden, daß die Verwendung eines Pflanzenfetts mit
a) einem Wassergehalt von höchstens 0,15 Gew%,
b) einem Gehalt an freien Fettsäuren von höchstens 0,15 Gew%,
c) einer Peroxidzahl nach Wheeler von höchstens 1,5 meq O₂/kg,
d) einem Steigschmelzpunkt von höchstens 45°C und
e) einer Jodzahl von höchstens 100, in dem
f) mindestens drei verschiedene an Glycerin gebundene Fettsäuren, ausgewählt aus
   Caprylsäure, Caprinsäure, Laurinsäure, Myristinsäure, Palmitinsäure, Stearinsäure, Ölsäure und Linolsäure, in einem Anteil von jeweils mindestens 5 % und gegebenenfalls weitere an Glycerin gebundene Fettsäuren in einem Anteil von jeweils höchstens 5 %, bezogen auf die Gesamtmasse der an Glycerin gebundenen Fettsäuren, vorkommen,
sowie von Gemischen solcher Pflanzenfette als Emoillient oder Konsistenzgeber in kosmetischen oder pharmazeutischen Präparaten die Nachteile des Standes der Technik nicht aufweist.

So sind diese Pflanzenfette in Lebensmittelqualität toxikologisch und ökotoxikologisch unbedenklich. Sie zeichnen sich durch eine hohe Lagerstabilität wie auch Oxidationsstabilität aus und sind somit geruchlich neutral und bringen auch keine toxikologisch bedenklichen Zersetzungsprodukte in die Endformulierung mit ein. Zusätzlich verleihen sie den kosmetischen bzw. pharmazeutischen Präparaten hervorragende taktile Eigenschaften, so daß diese Präparate beim Auftragen auf die Haut als sehr angenehm empfunden werden.

Diese erfindungsgemäßen Pflanzenfette können dabei allein als Emoillient eingesetzt werden. Sie können auch in den bestehenden Rezepturen die Fettbestandteile auf petrochemischer halbsynthetischer Basis ersetzen und auch als Konsistenzgeber wirken.

Ein weiterer Aspekt dieser Erfindung umfaßt kosmetische oder pharmazeutische Zusammensetzungen, die Pflanzenfette enthalten, bei denen
a) der Gehalt an freien Fettsäuren höchstens 0,15 Gew%,
b) die Peroxidzahl nach Wheeler höchstens 1,5 meq O₂/kg,
c) der Steigschmelzpunkt höchstens 45°C und
e) die Jodzahl höchstens 100 beträgt und in denen
f) mindestens drei verschiedene an Glycerin gebundene Fettsäuren, ausgewählt aus
   Caprylsäure, Caprinsäure, Laurinsäure, Myristinsäure, Palmitinsäure, Stearinsäure, Ölsäure und Linolsäure, in einem Anteil von jeweils mindestens 5 % und gegebenenfalls weitere an Glycerin gebundene Fettsäuren in einem Anteil von jeweils höchstens 5 %, bezogen auf die Gesamtmasse der an Glycerin gebundenen Fettsäuren, vorkommen.

Pflanzenfette für die erfindungsgemäße Verwendung enthalten üblicherweise verschiedene Triglyceride mit unterschiedlichen Steigschmelzpunkten.

Als besonders geeignet für die erfindungsgemäße Verwendung haben sich Pflanzenfette mit einem Wassergehalt von vorzugsweise höchstens 0,1 Gew.%, bevorzugter höchstens 0,05 Gew.%, (gemessen nach DGF-Einheitsmethoden C-III 13a), mit einem Gehalt an freien Fettsäuren von höchstens 0,1 Gew.%, bevorzugter höchstens 0,05 Gew.%, (gemessen nach DGF-Einheitsmethoden C-III 4), einer Peroxidzahl nach Wheeler von vorzugsweise höchstens 1 meq 0₂/kg, bevorzugter höchstens 0,3 meq 0₂/kg, (gemessen nach DGF-Einheitsmethoden C-VI 6a), einem Steigschmelzpunkt von vorzugsweise 15°C bis 45°C, bevorzugter zwischen 20°C und 38°C, am meisten bevorzugt zwischen 20°C und 30°C (gemessen nach DAB 10 V.611.2 Offene Kapillarmethode) und einer Jodzahl von vorzugsweise zwischen 2 und 100, bevorzugter zwischen 8 und 95 (gemessen nach DGF-Einheitsmethoden C-V 11d), erwiesen. Bevorzugt sind Pflanzenfette, bei denen der Anteil an weiteren an Glycerin gebundenen Fettsäuren unter 1% liegt, bezogen auf die Gesamtmasse der an Glycerin gebundenen Fettsäuren.

Vorzugsweise sind die Pflanzenfette in Alkohol weitgehend unlöslich.

Die erfindungsgemäßen Pflanzenfette können in einer Vielzahl von kosmetischen und pharmazeutischen Präparaten als Emoillient oder Konsistenzgeber verwendet werden. Beispiele hierfür sind im allgemeinen Öl-in-Wasser-Emulsionen oder Wasser-in-Öl-Emulsionen, insbesondere kosmetische Hautcremes und Lotionen, rückfettende Körperreinigungsmittel, Rasierschaum, Lippenstifte, kosmetische oder

medizinische Ölbäder, Zäpfchen, galenische Massen für pharmazeutische Produkte etc.

Je nach Anwendungsbereich können die erfindungsgemäßen Pflanzenfette in einer Menge von 1-99 Gew.%, bezogen auf die Gesamtmasse der kosmetischen oder pharmazeutischen Zusammensetzung enthalten sein. Darüber hinaus können noch weitere Fette oder Öle bis zu einem Gehalt von 50%, bezogen auf die Gesamtmasse aller Fett- und Ölkomponenten in diesen Zusammensetzungen vorhanden sein.

Weiterhin können die kosmetischen oder pharmazeutischen Zusammensetzungen je nach Anwendungsbereich die üblichen, dem Fachmann bekannten Zusatzstoffe, wie z.B. auch Konservierungsmittel, Wirkstoffe und Duftstoffe, in üblichen Mengen enthalten.

Ein Vorteil der erfindungsgemäß verwendeten Pflanzenfette liegt in ihrer Stabilität gegenüber Autoxidationsreaktionen. Daher kann in der Regel auch auf Antioxidantien verzichtet werden. Somit sind die pharmazeutischen und kosmetischen Präparate, die die erfindungsgemäßen Fettsäuren enthalten, vorzugsweise frei von Antioxidantien. Je nach Anwendungsbereich der Präparate und beigemengten Zusatzstoffen können aber auch Antioxidantien in einer Menge von höchstens 0,02 Gew.%, vorzugsweise höchstens 0,01 Gew.%, bezogen auf die Gesamtmasse der Zusammensetzung enthalten sein. Falls Antioxidantien zugesetzt werden, sind α-Tocopherol und/oder α-Tocopherolester, insbesondere α-Tocopherolacetat, bevorzugt.

Die vorliegende Erfindung wird nun anhand der folgenden Beispiele näher erläutert. Prozentangaben, sofern nicht anders angegeben, beziehen sich auf Gewicht.

Folgende Fette wurden bezüglich ihrer Fettsäureverteilung, den Gehalt an freien Fettsäuren, der Peroxidzahl, dem Wassergehalt, der Jodzahl und ihres Steigschmelzpunktes untersucht. Dabei wurden die Fettsäuren mit den bekannten Methoden der Gaschromatographie bestimmt. Der Wassergehalt wurde nach DGF-Einheitsmethoden C-III 13a, der Gehalt an freien Fettsäuren nach DGF-Einheitsmethoden C-III 4, die Peroxidzahl nach Wheeler gemäß DGF-Einheitsmethoden C-VI 6a, der Steigschmelzpunkt nach DAB 10 V.611.2 Offene Kapillarmethode und die Jodzahl nach DGF-Einheitsmethoden C-V 11d bestimmt.

Folgende Fette wurden dieser Beurteilung unterzogen:

### 1. Sojaöl raffiniert (Fa. Henry Lamotte) - kein Fett entsprechend der vorliegenden Erfindung

| Fettsäurezusammensetzung (HRGCC) | |
|---|---|
| Palmitinsäure (Hexadecansäure) | 8,7 % |
| Stearinsäure (Octadecansäure) | 4,3 % |
| Ölsäure (9-Octadecensäure) | 21,2 % |
| Linolsäure (9,12 Octadecadiensäure) | 56,6 % |
| Linolensäure 9,12,15 (Octadecatriensäure) | 7,7 % |
| Fettsäuren mit einer C-Zahl über C 18 | Rest |
| Freie Fettsäuren | 0,25 % |
| Peroxidzahl nach Wheeler | 0,5 meq0₂/kg |
| Wasser | 0,2 % |
| Jodzahl nach Wijs | 136 |
| Steigschmelzpunkt | -10 bis -17°C |

### 2. Olivenöl (Fa. Henry Lamotte) - kein Fett entsprechend der vorliegenden Erfindung

| Fettsäurezusammensetzung (HRGCC) | |
|---|---|
| Myristinsäure (Tetradecansäure) | 0,5 % |
| Palmitinsäure (Hexadecansäure) | 9,5 % |
| Stearinsäure (Octadecansäure) | 2,0 % |
| Ölsäure (9-Octadecensäure) | 81,0 % |
| Linolsäure (9,12 Octadecadiensäure) | 7,0 % |
| Freie Fettsäuren | 0,5 % |
| Peroxidzahl nach Wheeler | 0,8 meq 0₂/kg |
| Wasser | 0,2 % |
| Jodzahl nach Wijs | 80-88 |
| Steigschmelzpunkt | -3 bis 0°C |

### 3. Rüböl (Fa. O.&L. Sels) - kein Fett entsprechend der vorliegenden Erfindung

| Fettsäurezusammensetzung (HRGCC) | |
|---|---|
| Palmitinsäure (Hexadecansäure) | 2,5 - 4,5 % |
| Stearinsäure (Octadecansäure) | 0,5 - 2,5 % |
| Ölsäure (9-Octadecensäure) | 54 - 64 % |
| Linolsäure (9,12 Octadecadiensäure) | 17 - 24 % |
| Linolensäure 9,12,15 (Octadecatriensäure) | 7 - 11 % |
| Fettsäuren mit einer C-Zahl über C 18 | Rest % |
| Freie Fettsäuren | 0,15 % |
| Peroxidzahl nach Wheeler | 0,4 meq O₂/kg |
| Wasser | 0,2 % |
| Jodzahl nach Wijs | 110-120 |

### 4. Cremeol PS-17 (Fa. Arhuis Olie) - kein Fett entsprechend der vorliegenden Erfindung

| Fettsäurezusammensetzung (HRGCC) | |
|---|---|
| Fettsäuren mit einer C-Zahl unter C16 | 1,0 % |
| Palmitinsäure (Hexadecansäure) | 12,0 % |
| Stearinsäure (Octadecansäure) | 3,0 % |
| Ölsäure (9-Octadecensäure) | 72,0 % |
| Linolsäure (9,12 Octadecadiensäure) | 11,0 % |
| Fettsäuren mit einer C-Zahl über C 18 | 1,0 % |
| Freie Fettsäuren | 0,2 % |
| Peroxidzahl nach Wheeler | 0,5 meq O₂/kg |
| Wasser | 0,15 % |
| Jodzahl nach Wijs | 81-88 |
| Steigschmelzpunkt | 23-26°C |

### 5. Fett für die Verwendung entsprechend der vorliegenden Erfindung

| Fettsäurezusammensetzung (HRGCC) | |
|---|---|
| Caprylsäure (Octansäure) | 7,6 % |
| Caprinsäure (Decansäure) | 6,6 % |
| Laurinsäure (Dodecansäure) | 46,8 % |
| Myristinsäure (Tetradecansäure) | 17,7 % |
| Palmitinsäure (Hexadecansäure) | 9,0 % |
| Stearinsäure (Octadecansäure) | 2,6 % |
| Ölsäure (9-Octadecensäure) | 7,3 % |
| Linolsäure (9,12 Octadecadiensäure) | 1,9 % |
| Freie Fettsäuren | 0,02 % |
| Peroxidzahl nach Wheeler | 0 meq O₂/kg |
| Wasser | 0,02 % |
| Jodzahl nach Wijs | 8-11 |
| Steigschmelzpunkt | 23-26°C |

### 6. Fett für die Verwendung entsprechend der vorliegenden Erfindung

| Fettsäurezusammensetzung (HRGCC) | |
|---|---|
| Caprylsäure (Octansäure) | 7,2 % |
| Caprinsäure (Decansäure) | 6,5 % |
| Laurinsäure (Dodecansäure) | 47,4 % |
| Myristinsäure (Tetradecansäure) | 17,5 % |
| Palmitinsäure (Hexadecansäure) | 8,8 % |
| Stearinsäure (Octadecansäure) | 11,5 % |
| Ölsäure (9-Octadecensäure) | 1,1 % |
| Freie Fettsäuren | 0,02 % |
| Peroxidzahl nach Wheeler | 0 meq O₂/kg |
| Wasser | 0,02 % |
| Jodzahl nach Wijs | max. 2 |
| Steigschmelzpunkt | 34,5-37,0°C |

### 7. Fett für die Verwendung entsprechend der vorliegenden Erfindung

| Fettsäurezusammensetzung (HRGCC) | |
|---|---|
| Palmitinsäure (Hexadecansäure) | 10,9 % |
| Stearinsäure (Octadecansäure) | 4,7 % |
| Ölsäure (9-Octadecensäure) | 56,3 % |
| Linolsäure (9,12 Octadecadiensäure) | 26,4 % |
| Linolensäure 9,12,15 (Octadecatriensäure) | 0,8 % |
| Arachinsäure (Eicosansäure) | 0,3 % |
| Gadoleinsäure (Eicosensäure) | 0,2 % |
| Behensäure (Docosansäure) | 0,4 % |
| Freie Fettsäuren | 0,04 % |
| Peroxidzahl nach Wheeler | 0,25 meq O₂/kg |
| Wasser | 0,025 % |
| Jodzahl nach Wijs | 94-98 |
| Steigschmelzpunkt | 16-24°C |

### 8. Fett für die Verwendung entsprechend der vorliegenden Erfindung

| Fettsäurezusammensetzung (HRGCC) | |
|---|---|
| Palmitinsäure (Hexadecansäure) | 10,5 % |
| Stearinsäure (Octadecansäure) | 8,5 % |
| Ölsäure (9-Octadecensäure) | 74,0 % |
| Linolsäure (9,12 Octadecadiensäure) | 6,0 % |
| Arachinsäure (Eicosansäure) | 0,3 % |
| Gadoleinsäure (Eicosensäure) | 0,3 % |
| Behensäure (Docosansäure) | 0,4 % |
| Freie Fettsäuren | 0,04 % |
| Peroxidzahl nach Wheeler | 0,2 meq O₂/kg |
| Wasser | 0,02 % |
| Jodzahl nach Wijs | 70-75 |
| Steigschmelzpunkt | 33-35°C |

Zusätzlich zu diesen meßbaren Kriterien wurde die geruchliche Eignung der Pflanzenfette durch ein Testpanel von 10 Personen, die mit der geruchlichen Beurteilung von kosmetischen Präparaten vertraut sind, ermittelt. Bei diesem Test wurden die Fette jeweils in einer Menge von 0,5 g auf ein Hautareal im Unterarmbereich appliziert und sofort nach der Applikation, dann nach 1 Stunde, nach 4 Stunden und nach 8 Stunden geruchlich beurteilt. Die Pflanzenfette 1-4 entwickelten nach dem Auftragen auf die Haut einen unangenehmen fettigen und zum Teil ranzigen Beigeruch. Die Fette 5-8, die die erfindungswesentlichen analytischen Daten erfüllen, entsprechen dagegen in hervorragender Weise den geruchlichen Anforderungen.

Mit den pflanzlichen Fetten Nr. 5, 6, 7 und 8 wurden Öl-in-Wasser-Emulsionen nach den Beispielen 1-4 hergestellt und unter geruchlichen Aspekten mit einer Emulsion Beispiel 5 verglichen, die Paraffinöl als Emoillient enthält. Die Öl-in-Wasser-Emulsionen wurden wie folgt hergestellt:

Axol C62, D-Panthenol 75%ig, Glycerin 87%ig wurde in 1/3 der Wassermenge auf 70°C erwärmt und dabei gerührt. Danach wurde Keltrol BT in Baysilonöl M 100 dispergiert und unter Rühren in die Wasserphase gegeben, dann wurde die Restmenge Wasser zugefügt.

Die Fettphase, bestehend aus dem erfindungsgemäßen Pflanzenfett bzw. Paraffinöl und Miglyol, Jojobaöl, Avocadoöl, Prisorine 2036, Vitamin E Acetat und Dehymuls HRE 7 wurde auf ca. 5°C über den Schmelzpunkt dieser Phase erwärmt und Synthalen K darin dispergiert und unter Rühren der Wasserphase zugesetzt.

Nach gutem Durchmischen wurde Phenonip und Propylenglycol dem Ansatz unter Rühren zugemischt.

Nachdem der Ansatz homogen erschien, wurde Kalilauge 25%ig zugefügt, mit einem geeigneten Homogenisator homogenisiert und bis zu einer Temperatur von ca. 30°C gerührt.

Die Zusammensetzung dieser Öl-in-Wasser-Emulsionen wird in Tabelle 1 wiedergegeben.

**Tabelle 1**

| Rohstoffe | Bsp. 1 Gew.% | Bsp. 2 Gew.% | Bsp. 3 Gew.% | Bsp. 4 Gew.% | Bsp. 5 Gew.% |
|---|---|---|---|---|---|
| Wasser | 74,74 | 74,74 | 74,74 | 74,74 | 74,74 |
| D-Panthenol 75%ig | 0,13 | 0,13 | 0,13 | 0,13 | 0,13 |
| Axol C 62 | 3,00 | 3,00 | 3,00 | 3,00 | 3,00 |
| Keltrol BT | 0,08 | 0,08 | 0,08 | 0,08 | 0,08 |
| Synthalen K | 0,34 | 0,34 | 0,34 | 0,34 | 0,34 |
| Miglyol 812 | | | | | 4,00 |
| Fett Nr. 5 | 11,00 | | | | |
| Fett Nr. 6 | | 11,00 | | | |
| Fett Nr. 7 | | | 11,00 | | |
| Fett Nr. 8 | | | | 11,00 | |
| Paraffinöl | | | | | 7,00 |
| Jojobaöl | 1,00 | 1,00 | 1,00 | 1,00 | 1,00 |
| Phenonip | 1,10 | 1,10 | 1,10 | 1,10 | 1,10 |
| Propylenglycol | 3,00 | 3,00 | 3,00 | 3,00 | 3,00 |
| Avocadoöl | 1,00 | 1,00 | 1,00 | 1,00 | 1,00 |
| Baysilonöl M 100 | 0,50 | 0,50 | 0,50 | 0,50 | 0,50 |
| Prisorine 2036 | 1,00 | 1,00 | 1,00 | 1,00 | 1,00 |
| Vitamin E-Acetat | 0,01 | 0,01 | 0,01 | 0,01 | 0,01 |
| Kalilauge 25%ig | 0,60 | 0,60 | 0,60 | 0,60 | 0,60 |
| Dehymuls HRE 7 | 0,50 | 0,50 | 0,50 | 0,50 | 0,50 |
| Glycerin 87%ig DAB | 2,00 | 2,00 | 2,00 | 2,00 | 2,00 |

| Kennzahlen: | | | | | |
|---|---|---|---|---|---|
| pH-Wert (elektrometrisch) | 6,1 | 6,0 | 6,3 | 6,1 | 6,1 |
| Viskosität (mpas) bei 20°C (Viskotester RV 20 der Fa. Haake Meßkörper MV DIN Stufe 4) | 3200 | 3200 | 2500 | 4200 | 2200 |

Geruchlich wurden die Präparate gemäß den Beispielen 1-5 nach 3 Monaten und nach 12 Monaten beurteilt. Für diesen Test wurden die bei 20°C und bei 40°C gelagerten Präparate herangezogen. Von einem ausgebildeten Parfumeur und einer Testpanele von 6 Personen wurden die Emulsionen nach diesen Zeiträumen als geruchlich gut bis sehr gut beurteilt.

Die Anwendungseigenschaften hinsichtlich Hautgefühl nach dem Auftragen, Aussehen der Haut nach dem Auftragen, Geschwindigkeit der Absorption durch die Haut und Pflegegefühl nach 1 Std. und 4 Std. wurden ebenfalls als ausgezeichnet bezeichnet.

Hinsichtlich Geruch, anwendungstechnischer Eigenschaften und Lagerstabilität wurden keine Unterschiede zwischen den Emulsionen mit den erfindungsgemäßen Pflanzenfetten und der Emulsion mit Paraffinöl (Beispiel 5) festgestellt.

Eine Phasentrennung wurde nach 3 Monaten Lagerung bei 40°C, bei 4°C, bei -18°C und bei 20°C nicht beobachtet. Bei einer 12monatigen Lagerung bei 20°C wurde ebenfalls keine Phasentrennung festgestellt.

In den Öl-in-Wasser-Emulsionen der Beispiele 6-8 wurden nur die erfindungsgemäßen Pflanzenfette, d.h. ohne andere Fettkomponenten eingesetzt. Ansonsten wurden die Emulsionen analog zu den Beispielen 1-5 hergestellt. Die Zusammensetzung dieser Emulsionen sowie die analytischen Daten sind in Tabelle 2 wiedergegeben.

**Tabelle 2**

| Rohstoffe | Bsp. 6 Gew.% | Bsp. 7 Gew.% | Bsp. 8 Gew.% |
|---|---|---|---|
| Wasser | 79,665 | 79,665 | 79,665 |
| D-Panthenol 75%ig | 0,130 | 0,130 | 0,130 |
| Axol C 62 | 3,000 | 3,000 | 3,000 |
| Keltrol BT | 0,100 | 0,100 | 0,100 |
| Synthalen K | 0,280 | 0,280 | 0,280 |
| Phenonip | 0,900 | 0,900 | 0,900 |
| Vitamin E-Acetat | 0,010 | 0,010 | 0,010 |
| Kalilauge 50%ig | 0,215 | 0,215 | 0,215 |
| Prisorine 3700 | 0,700 | 0,700 | 0,700 |
| Glycerin 87% | 3,000 | 3,000 | 3,000 |
| Fett Nr. 7 | 12,000 | | 6,000 |
| Fett Nr. 5 | | 12,000 | |
| Fett Nr. 8 | | | 6,000 |

| Kennzahlen: | | | |
|---|---|---|---|
| pH-Wert (elektrometrisch) | 6,1 | 6,1 | 6,0 |
| Viskosität (mpas) bei 20°C (Viskotester RV 20 der Fa. Haake Messkörper MV DIN Stufe 4) | 1270 | 1280 | 1450 |

Die Präparate, die gemäß den Beispielen 6-8 gefertigt wurden, erfüllen ebenfalls die geruchlichen und anwendungstechnischen Anforderungen. Die Lagerstabilität bei 40°C, bei 4°C, bei -18°C und bei 20°C ist ebenfalls ausgezeichnet.

Beispiel 9 betrifft eine Wasser-in-Öl-Emulsion mit einem erfindungsgemäßen Pflanzenfett, das mit einer Wasser-in-Öl-Emulsion des Beispiels 10, das als Emoillient eine Mischung aus Paraffinöl, einem synthetischen Triglycerid (Miglyol 812) und einer Mischung eines Paraffinöls, Lanolin und Lanolinalkohol (Amerchol C) enthält, verglichen wird. Die Wasser-in-Öl-Emulsionen wurden dabei wie folgt hergestellt:

Die Fettphase, bestehend aus Protegin, Neo PCL W/0, Fett Nr. 5 bzw. statt Fett Nr. 5 die Komponenten Weissöl 5 Grad E DAB 8, Miglyol 812 und Amerchol wurde auf 80°C erwärmt.

Das auf 90°C erhitzte Wasser und das darin gelöste Nipagin, Nipasol Glycerin 87%ig, Bittersalz und Hydroviton wurde unter Rühren in die Fettphase gegeben.

Nach Rühren und Kühlen auf 45°C wurde das Vitamin E Acetat zugegeben und der Ansatz bis auf ca. 30°C unter Rühren abgekühlt.

Die Zusammensetzungen der Wasser-in-Öl-Emulsionen sind in Tabelle 3 wiedergegeben.

**Tabelle 3**

| Rohstoffe | Beispiel 9 Gew.% | Beispiel 10 Gew.% |
|---|---|---|
| Wasser | 57,486 | 57,486 |
| Nipagin | 0,150 | 0,150 |
| Nipasol | 0,050 | 0,050 |
| Glycerin 87% | 2,000 | 2,000 |
| Bittersalz | 0,300 | 0,300 |
| Hydroviton | 2,000 | 2,000 |
| Protegin | 20,000 | 20,000 |
| Neo PCL W/0 E 2/066255 | 5,000 | 5,000 |
| Fett Nr. 5 | 13,000 | |
| Weissöl 5 Grad E DAB 8 | | 7,000 |
| Miglyol 812 | | 5,000 |
| Amerchol C | | 1,000 |
| Vitamin A Acetat (1,5 Mio I.E.) | 0,013 | 0,013 |
| Vitamin E Acetat | 0,001 | 0,001 |

Die Wasser-in-Öl-Emulsionen besitzen eine salbenartige Konsistenz.

Hinsichtlich Geruch, anwendungstechnischer Eigenschaften und Lagerstabilität wurden keine Unterschiede zwischen dem Beispiel 9 mit dem erfindungsgemäßen Pflanzenfett und dem Beispiel 10 festgestellt.

In dem Beispiel 11 wird ein Badeöl mit einem erfindungsgemäßen Pflanzenfett hergestellt und mit dem Beispiel 12, das als Emoillient ein Paraffinöl und ein synthetisches Triglycerid (Miglyol 812) enthält, verglichen. Die in Tabelle 4 angegebenen Komponenten wurden in einem Ansatzbehälter bis zur optischen Klarheit bei ca. 25°C durch Rühren vermischt. Die Zusammensetzung der Badeöle ist in Tabelle 4 wiedergegeben.

**Tabelle 4**

| Rohstoffe | Beispiel 11 Gew.% | Beispiel 12 Gew.% |
|---|---|---|
| PCL Liquid | 10,00 | 10,00 |
| Estol 3768 SMIS | 4,00 | 4,00 |
| Hostaphat KL 340 N | 5,00 | 5,00 |
| Parfüm | 3,50 | 3,50 |
| Fett Nr. 7 | 77,50 | |
| Miglyol 812 | | 20,00 |
| Paraffinöl | | 57,50 |

Hinsichtlich Geruch, anwendungstechnische Eigenschaften und Lagerstabilität wurden keine Unterschiede zwischen dem Beispiel 11 mit dem erfindungsgemäßen Pflanzenfett und dem Beispiel 12 festgestellt.

In dem Beispiel 13 wird ein medizinischer Erkältungsstift mit einem erfindungsgemäßen Pflanzenfett hergestellt mit dem Produkt des Beispiels 14, das als Emoillient eine Mischung aus synthetischen Triglyceriden enthält, verglichen. Die Erkältungsstifte wurden dabei wie folgt hergestellt: Die In Tabelle 5 angegebenen Komponenten wurden in einem Ansatzbehälter bis zur optischen Klarheit bei ca. 55°C durch Rühren vermischt.

Danach wurde die Masse in Stifthülsen gegossen, die Stifthülsen mit Schraubverschlüssen verschraubt und um 180°C gedreht und auf ca. 20°C abgekühlt.

Die Zusammensetzung der Erkältungsstifte ist in Tabelle 5 wiedergegeben.

**Tabelle 5**

| Rohstoffe | Beispiel 13 Gew.% | Beispiel 14 Gew.% |
|---|---|---|
| Wirkstoffmischung | 9,75 | 9,75 |
| Lanette 0 | 35,00 | 35,00 |
| Cutina HR | 4,00 | 4,00 |
| Cutina GMS | 5,00 | 5,00 |
| Fett Nr. 6 | 46,25 | |
| Novata B | | 46,25 |

Hinsichtlich Geruch, anwendungstechnischer Eigenschaften und Lagerstabilität wurden keine Unterschiede zwischen dem Beispiel 13 mit dem erfindungsgemäßen Pflanzenfett und dem Beispiel 14 festgestellt.

Die Vergleichsbeispiele zeigen, daß die aufgrund ihrer toxikologischen und ökotoxikologischen Eigenschaften bedenklichen Paraffinöle bzw. synthetischen Triglyceride durch die erfindungsgemäßen Pflanzenfette ersetzt werden können, ohne daß sich dadurch die gewünschten geruchlichen und anwendungstechnischen Eigenschaften oder die Lagerstabilität dabei verschlechtern.

Die in den Beispielen verwendeten Rohstoffe entsprechen folgenden chemischen Charakterisierungen:
- D-Panthenol 75%ig: 75%ige Lösung von D-Pantothenylalkohol
- Protegin: Kombination nichtionogener Emulgatoren mit Sterinen, aliphatischen Alkoholen und Kohlenwasserstoffen
- Hydroviton: glycerinhaltiges Feuchthaltemittel
- Weissöl 5 Grad E DAB 8: Mischung gereinigter, gesättigter Kohlenwasserstoffe
- Neo PCL W/O: Adsorptionsbase für W/O Emulsionen
- Amerchol C: Mischung aus Petrolatum, Lanolin und Lanolinalkohol
- Axol C62: teilneutralisierter Zitronensäureester von molekulardestillierten Stearin-, Palmitinsäuremonoglyceriden pflanzlichen Ursprungs
- Keltrol BT: Xanthan Gum - Polysaccharid
- Synthalen K: Polyacrylsäure, Homopolymer
- Miglyol 812: Capryl/Caprinsäuretriglyceride
- Pionier 4656 P: Paraffinium liquidum, ein hochausraffiniertes, hochsiedendes Mineralölprodukt mit einer Reinheit nach DAB 10
- Jojobaöl Phenonip: kaltgepreßtes Jojobaöl DAC 1986 Mischung von Alkylestern der 4-Hydroxybenzoesäure und 2-Phenoxyäthanol
- Propylenglycol: 1,2 Propylenglycol USP
- Avocadoöl: raffiniertes Avocadoöl DAC 1986
- Baysilonöl M 1 00: Polydimethylsiloxan
- Prisorine 2036: 2-Ethylhexylisostearat
- Prisorine PG3DI 3700: Triglyceryl-3-diisostearate
- Vitamin E Acetat: DL -alpha- Tocopherylacetat
- Vitamin A Acetat (1,5 Mio I.E.): Retinylacetat gelöst in Erdnußöl
- Kalilauge 25%ig: 25%ige wäßrige Lösung von Kaliumhydroxid
- Kalilauge 50%ig: 50%ige wäßrige Lösung von Kaliumhydroxid
- Dehymuls HRE 7: Hydriertes Rizinusöl mit 7 Mol EO
- Glycerin 87%: 1,2,3 Propantriol 87%ig in Wasser nach DAB 10
- Lanette O: Mischung aus Cetyl- und Stearylalkohol
- Cutina HR: Gehärtetes Rizinusöl
- Cutina GMS: Glycerinmonostearat
- Novata B: Gemisch von Mono-, Di- und Triglyceriden gesättigter Fettsäuren
- Texapon N 70: Natriumlaurylethersulfat
- Tegobetain F 50: Cocamidopropyl Betain
- Plantaren 1200: Lauryl Polyglucose
- Cetiol HE: PEG-7 Glycerylcocoate
- Jaguar C 162: Hydroxypropyl Guar Hydroxypropyltriammonium Chloride
- Hostaphat KL 340 N: Trilaureth-4 Phosphate
- Estol 3768 SMIS: Sorbitan Monoisostearat
- PCL Liquid: Gemisch alkylverzweigter Fettsäureester
- Nipagin: Methylparaben
- Nipasol: Propylparaben

Bei der verwendeten Wasserqualität handelt es sich um enthärtetes Wasser mit einer Leitfähigkeit von < 5 micro Siemens.

## Patentansprüche

1. Verwendung eines Pflanzenfetts mit
a) einem Wassergehalt von höchstens 0,15 Gew%,
b) einem Gehalt an freien Fettsäuren von höchstens 0,15 Gew%,
c) einer Peroxidzahl nach Wheeler von höchstens 1,5 meq O₂/kg,
d) einem Steigschmelzpunkt von höchstens 45°C und
e) einer Jodzahl von höchstens 100, in dem
f) mindestens drei verschiedene an Glycerin gebundene Fettsäuren, ausgewählt aus
Caprylsäure, Caprinsäure, Laurinsäure, Myristinsäure, Palmitinsäure, Stearinsäure, Ölsäure und Linolsäure, in einem Anteil von jeweils mindestens 5 % und gegebenenfalls weitere an Glycerin gebundene Fettsäuren in einem Anteil von jeweils höchstens 5 %, bezogen auf die Gesamtmasse der an Glycerin gebundenen Fettsäuren, vorkommen,
sowie von Gemischen solcher Pflanzenfette als Emoillient oder Konsistenzgeber in kosmetischen oder pharmazeutischen Präparaten.

2. Verwendung nach Anspruch 1
**dadurch gekennzeichnet**,
daß das Pflanzenfett Triglyceride mit unterschiedlichen Steigschmelzpunkten enthält.

3. Verwendung nach einem der vorstehenden Ansprüche
**dadurch gekennzeichnet**,
daß der Wassergehalt höchstens 0,1 Gew% beträgt.

4. Verwendung nach einem der vorstehenden Ansprüche
**dadurch gekennzeichnet**,
daß der Gehalt an freien Fettsäuren höchstens 0,1 Gew% beträgt.

5. Verwendung nach einem der vorstehenden Ansprüche
**dadurch gekennzeichnet**,
daß die Peroxidzahl nach Wheeler höchstens 1 meq O₂/kg beträgt.

6. Verwendung nach einem der vorstehenden Ansprüche
**dadurch gekennzeichnet**,
daß die Jodzahl zwischen 5 und 95 liegt.

7. Verwendung nach einem der vorstehenden Ansprüche
**dadurch gekennzeichnet**,
daß der Steigschmelzpunkt zwischen 20°C und 38°C liegt.

8. Kosmetische oder pharmazeutische Zusammensetzung, die Pflanzenfette enthält, bei denen
a) der Gehalt an freien Fettsäuren höchstens 0,15 Gew%,
b) die Peroxidzahl nach Wheeler höchstens 1,5 meq O₂/kg,
c) der Steigschmelzpunkt höchstens 45°C und
e) die Jodzahl höchstens 100 beträgt und in denen
f) mindestens drei verschiedene an Glycerin gebundene Fettsäuren, ausgewählt aus
Caprylsäure, Caprinsäure, Laurinsäure, Myristinsäure, Palmitinsäure, Stearinsäure, Ölsäure und Linolsäure, in einem Anteil von jeweils mindestens 5 % und gegebenenfalls weitere an Glycerin gebundene Fettsäuren in einem Anteil von jeweils höchstens 5 %, bezogen auf die Gesamtmasse der an Glycerin gebundenen Fettsäuren, vorkommen.

9. Zusammensetzung nach Anspruch 8
**dadurch gekennzeichnet**,
daß der Gehalt an freien Fettsäuren in diesen Fetten höchstens 0,1 Gew% beträgt.

10. Zusammensetzung nach einem der Ansprüche 8 oder 9
**dadurch gekennzeichnet**,
daß die Peroxidzahl nach Wheeler dieser Fette höchstens 1 meq O₂/kg beträgt.

11. Zusammensetzung nach einem der Ansprüche 8 - 10
**dadurch gekennzeichnet**,
daß die Jodzahl dieser Fette zwischen 5 und 95 liegt.

12. Zusammensetzung nach einem der Ansprüche 8 - 11
**dadurch gekennzeichnet**,
daß der Steigschmelzpunkt dieser Fette zwischen 20°C und 38°C liegt.

13. Zusammensetzung nach einem der Ansprüche 8 - 12
**dadurch gekennzeichnet**,
daß diese Pflanzenfette in einer Menge von 1 bis 99 Gew% bezogen auf die Gesamtmasse der Zusammensetzung enthalten sind.

14. Zusammensetzung nach einem der Ansprüche 8 - 13
**dadurch gekennzeichnet**,
daß noch weitere Fette oder Öle bis zu einem Gehalt von 50% bezogen auf die Gesamtmasse aller Fett- und Ölkomponenten der Zusammensetzung enthalten sind.

15. Zusammensetzung nach einem der Ansprüche 8 - 14
**dadurch gekennzeichnet**,
daß bis zu 0,02 Gew% Antioxidantien bezogen auf die Gesamtmasse der Zusammensetzung enthalten sind.

16. Zusammensetzung nach Anspruch 15
**dadurch gekennzeichnet**,
daß bis zu 0,01 Gew% Antioxidantien bezogen auf die Gesamtmasse der Zusammensetzung enthalten sind.

17. Zusammensetzung nach einem der Ansprüche 8 - 14
**dadurch gekennzeichnet**,
daß die Zusammensetzung frei von Antioxidantien ist.
